# EUROPEAN PATENT SPECIFICATION

(11) **EP 0 528 469 B1**
(45) Date of publication and mention of the grant of the patent: **03.07.1996**
(21) Application number: 92202294.2
(22) Date of filing: 23.07.1992
(51) Int. Cl.: C12N 15/13, C12N 1/21, C07K 16/24, A61K 39/395

(54) **Recombinant DNA-molecule for the expression of the FV-fragment of an antibody**
Rekombinantes DNS-Molekül zur Expression von FV-Fragmenten eines Antikörpers
Molécule d'ADN recombinant, pour l'expression de fragments FV d'un anticorps

(30) Priority: 23.07.1991 NL 9101290
(43) Date of publication of application: 24.02.1993
(73) Proprietor: Stichting REGA V.Z.W., B-3000 Leuven (BE)
(72) Inventor: Billiau, Alfons Jozef Denis Alida, B-3210 Linden (BE); Froyen, Guido Frans Valentius, B-3020 Herent (BE)
(74) Representative: Hoijtink, Reinoud

(56) References cited:
- EP-A- 0 368 684
- NATURE. vol. 341, 12 October 1989, LONDON GB pages 544 - 546 E.S. WARD ET AL. 'Binding activities of a repertoire of single immunoglobin variable domains secreted from Escherichia coli'
- MICROBIOLOGY AND IMMUNOLOGY vol. 31, no. 8, 1987, pages 809 - 820 T. JITSUKAWA ET AL. 'Characterization of murine monoclonal antibodies to human interferon-gamma and their application for sandwich enzyme-linked immunosorbent assay'
- MOLECULAR IMMUNOLOGY vol. 27, no. 8, 1990, pages 745 - 750 E. DEPLA EN M. DE LEY 'Monoclonal antibodies against the human interferon-gamma receptor(s)'
- BIOTECHNOLOGY vol. 9, no. 6, 1 June 1991, NEW YORK US pages 545 - 551 A. PLÜCKTHUN 'Antibody engineering: Advances from the use of Escherichia coli expression systems'
- J.Biol.Chem 268:2577 (Schodin et al.93)
- Bio Technology 10:617 (Borrebaeck et al 1992)
- Mol. Immunol. 30:805 (Froyen et al; 1993)

## Description

The present invention relates to a recombinant DNA-molecule for the expression of a functional Fv-fragment of a desired antibody, directed toward interferon. The invention further relates to a recombinant *E.coli-strain*, comprising the recombinant DNA-molecule, a method for preparing the recombinant DNA-molecule, and a method of preparing a recombinant Fv-fragment of a desired anti-interferon antibody. The invention relates in particular to a recombinant DNA-molecule for the expression of a functional Fv-fragment of a anti-human-interferon-Γ (anti-HuIFN-Γ) antibody. The invention also relates to biological active preparations comprising said Fv-fragments and the use of said biological active preparations.

Immunoglobulins (antibodies) are glycoproteins present in the blood and bodyfluids of higher animal species. They recognize and bind certain categories of substances that are foreign to the body (antigens) and induce effector functions that eliminate or neutralize these antigens and their carriers (viruses, bacteria, cancer cells). An immunoglobulin comprises two identical light and two also identical heavy polypeptide chains (called L- and H-chains respectively), that are linked by means of disulphide bridges. The sequences of the N-terminal ends of the four chains are very variable and together responsible for the binding of the antigen. The variable regions therefore determine the specificity of the antibody. The C-terminal part of the chains has however a relatively constant structure and determines the nature of the effector functions, such as complement activation, antibody dependent cellular cytotoxicity and others.

Interferon-Γ (IFN-Γ) is a glycoprotein that is produced by T-cells in response to antigenic or mitogenic stimuli. In addition to its antiviral activity, interferon-Γ can also show strong immunoregulatory effects making it an important factor in the defense mechanism against bacteria, viruses and other antigens, but also in auto-immune diseases and cancer. The contribution of IFN-Γ in these pathological reactions can however also be harmful for the host.

Animal model studies have clarified that monoclonal antibodies against interferon-Γ have a positive effect on endotoxic shock, local inflammation, cerebral malaria and auto-immune arthritis. The monoclonal antibodies against interferon-Γ could therefore possibly also be used for the treatment of related human diseases.

Because of the ongoing development of hybridoma techniques a great number of monoclonal antibodies has become available that can be applied for the treatment of diseases. From numerous studies it is however found that the administration of xenogenic antibodies generates an immunoglobulin response in the recipient. The administration of human monoclonal antibodies could reduce or preclude such anti-immunoglobulin response. The production of those human monoclonal antibodies encounters ethical as well as practical objections. To avoid the above problems it has been tried recently to strongly reduce the unwanted anti-immunoglobulin response by means of in vitro manipulation of the immunoglobulin genes, such as chimerization or humanization. Important results have already been achieved herewith.

It has now been found that the Fv-fragment of a antibody directed towards human interferon-Γ has a affinity for interferon-Γ and is capable of neutralizing the biological activity thereof. It is expected that with the use of such a Fv-fragment the anti-immunoglobin response can also be reduced. This is caused by the fact that the immunogenic C-regions are removed form the antibody, while the antigen binding properties of the V-regions are maintained.

The present invention provides a recombinant DNA-molecule for expressing a functional Fv-fragment of a desired antibody directed towards human interferon-Γ. The recombinant DNA-molecule therefore comprises a homologous promoter sequence, a homologous terminator sequence, and a dicistronic heterlogous DNA-sequence, the cistrons of which encode the variable part of a heavy chain (V_{H}) of the desired antibody and the variable part of a light chain (V_{K}) of a desired antibody, respectively. Such a recombinant DNa-molecule provides the necessary simultaneous production of the genes of the variable regions of the light and heavy chain. Both variabel genes are preferably controled by the lac-promoter. This promoter can be inhibited of activated according to need. Thus it can be avoided that the Fv-fragment, which might be toxic to *E-coli* is produced at a undesirable moment. It is of course also possible to use other inducible promoters. Both variable genes are preferably preceded by a signal peptide sequence of *pel*B, trough which both V-regions are secreted into the peri plasm. It is also possible to connect both variable regions by means of a linker. Said linker preferably has the sequence according to formule Ic. In this case the signal peptide sequence is only comprised upstream from the cistron for the heavy chain.

Downstream of the cistron for the light chain a DNA-sequence encoding a small polypeptide, e.g. the TAG1-polypeptide, is preferably comprised. This polypeptide is used for detecting the Fv-fragment by means of a antibody directed towards the TAG1-polypeptide. The present invention will be further illustrated by the following examples, which are not intended to be limiting to the scope of the invention.

### EXAMPLE 1

### Isolation and amplification of the V_{H}- and V_{K}-fragment of anti-HuIFN-Γ.

The nucleotidesequence of both the 5'- and the 3'-ends of the V-regions of immunoglobulins is strongly conserved. Because of that it is possible to define oligonucleotides capable of amplifying the V_{H}- and V_{K}-regions of practically every mouse antibody (see Orlandi et al. (1989). Proc. Natl. Acad. SCI. USA 86: 3833-3837). The primers for the amplification of the V_{H}-regions are: VH1BACK : 5' AGGTSMARCTGCAGSAGTCWGG 3' 
VH1BACK : 5' AGGTSMARCTGCAGSAGTCWGG 3'
PstI
wherein : S = C or G
M = A or C
R = A or G
W = A or T 
VH1FOR : 5' TGAGGAGACGGTGACCGTGGTCCCTTGGCCCC 3'
BstEII
 The primers for the amplification of V_{K}-regions are: 
VK2BACK : 5' GACATCGAGCTCACCCAGTCTCCA 3'
SacI
 
VK2FOR : 5' GTTTGATCTCGAGCTTGGTGCC 3'
XhoI
 By the introduction of restriction sites in the amplified DNA-fragment the cloning of the fragments is considerably simplified.

For the amplification of 1 µg mRNA of D9D10 the first cDNA strand was synthesized in a way well known in the art. For this 100 pmol random hexanucleotide was used as a primer because after PCR a more specific DNA-fragment was obtained therewith than when a PCR-oligonucleotide was used for the first strand synthesis. The amplification of the desired fragment was performed on the reaction product of the first strand cDNA synthesis with the two specific primers in a 50 µl reaction mixture comprising 10 mM Tris-HCl pH 8.3, 50 mM KCl, 1.5 mM MgCl₂, 0.1 mg/ml gelatine, 0.25 mM dNTP, 25 pmol per oligonucleotide primer and 2.5 U Taq-polymerase . The reaction mixture was covered with 2 drops paraffine oil and subjected to 30 to 35 cycles of 30 sec. 95°C (denaturation), 35 sec. 61°C (annealing) and 25 sec. 72°C (filling-in reaction) with a last filling-in of 5 min. at 72°C. For performing the PCR a programmable thermic block was used (PCH-2; Techne, Cambridge, GB). Electroforesis of 5 µl of the reaction mixture on a 2% agarose gel showed the degree of purity and the yield of the amplified DNA-fragment. The other 45 µl were subjected to chloroform extraction (400 µl) after which the DNA was precipitated with NaA-c/ethanol. On this the desired reactions with the restriction enzymes were subsequently performed.

### EXAMPLE 2

### Construction of an expression vector for Fv-fragments of anti-HuIFN-Γ antibody.

For the construction of an expression vector for the Fv-fragments of anti-HuIFN-Γ the vector pSW1-VHD1.3-VKD1.3-TAG1 was used as described by Ward et al. (1989) Nature 341: 544-546. The V_{H}-domain of the antibody D1.3 was eliminated out of the vector by digestion with PstI and BstEII and replaced by the V_{H}-fragment of an anti-human-interferon-Γ of the hybridoma cell line D9D10, according to formula Ia. The fragment was obtained in sufficient amounts as described in example 1. The V_{K}-domain of the antibody D1.3 was then replaced by a SacI-XhoI-fragment according to formula Ib comprising the V_{K}-region of anti-HuIFN-Γ. The expression vector thus obtained was named pSW1-Fv-anti-HuIFN-Γ.

The nucleotide sequences of the V_{H}- and V_{K}-regions respectively (formula Ia and Ib respectively) of the D9D10 antibody are represented in the accompanying formula form. The parts in bold represent the primers used in the PCR. The underlined parts represent the restriction sites.

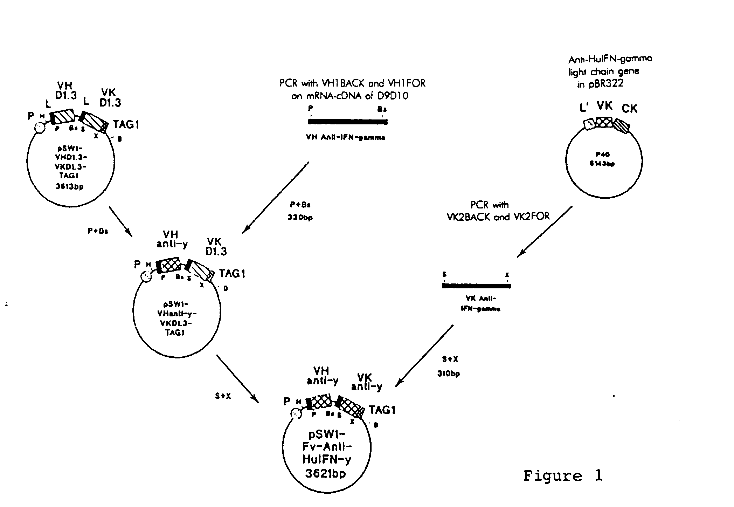

The strategy of construction is schematically represented in figure 7.

### EXAMPLE 3

### Transformation of E. coli (DH5α)

Plasmid DNA was brought into E. coli cells according to the CaCl₂ method of Mandel et al. ((1970) J. Mol. Biol. 53:159-162) except for the difference that only one heat shock was administered (instead of five) namely 20 sec. at 37°C for a 50 µl reaction, 45 sec at 37°C for a 100 µl reaction.

E. coli DH5α cells (supE44, hsdR17(rₖ-,mₖ+), recA1, endA1, gyrA96, thi-1, relA1, del(argF-laczya)U169, φ80d-lacZdelM15) were spread out after transformation in LB-medium (1% Bacto-trypton; 0.5% yeast extract and 0.8% softagar) on a solid LB-plate (1.5% agar) containing 100µg/ml amplicillin, 0.02% X-gal en 0.25 mM IPTG. They were incubated overnight at 37°C. In this manner clone P330 was obtained.

### EXAMPLE 4

### Expression of the Fv-fragment of anti-HuIFN-Γ.

The bacterial clone comprising the expression vector for the production of the Fv of the anti-HuIFN-Γ-antibody was grown for 16 hours at 37°C in LB-medium containing 100 µM ampicillin and 1% glucose. Glucose suppresses the lacpromotor which prevents the production of antibody fragments. Then the cells were washed twice in 50 mM NaCl and resuspended in LB-medium containing 100 µM ampicilline and 0.1-1 mM IPTG for induction of the promotor. This suspension was incubated for 2 to 20 hours in a shaking incubator at 37°C. The cells were then isolated by centrifugation (7000 rpm, 4°C) and the supernatant tested in an ELISA (see example 8) are stored at 4°C.

The periplasma was isolated as described by Skerra et al. (1988) Science 240: 1038-1041. After induction with IPTG the cells were precipitated by centrifugation, resuspended in TES-buffer (0.2 M Tris-HCl pH 8.0; 0.5 mM EDTA; 0.5M sucrose; 10 ml/l culture) and subjected to an osmotic shock by addition of a 1/4 dilution of TES-buffer in water (15 ml/l culture). After 30 minutes on ice the suspension was centrifuged by 8000 rpm for 10 minutes at 4°C. The supernatant was centrifuged again at 20,000 rpm for 30 minutes to remove the remaining celdebris. The supernatant was directly tested, stored at 4°C or frozen at -20°C.

### EXAMPLE 5

### Detection of the Fv-fragment of anti-HuIFN-Γ

The binding of the Fv-fragment to the human IFN-Γ molecule was tested in an ELISA. The wells of a 96-well "MaxiSorp Nunc-Immuno Plate" were incubated overnight at 4°C with 2 µg/ml recombinant HuIFN-Γ (Bioferon, Laupheim, Germany) in 100 µl of a 50 mH Tris-HCl pH 8.5 and 10 mM NaCl buffer. The stock solution of recombinant HuIFN-Γ was dissolved in 50 mM natrium phosphate and 100 mM ammonium acetate buffer with 12.5 mg/ml sucrose, to a concentration of 0.7 mg/ml pure proteine.

The culture plate was decanted and to every well 250 µl blocker (PBS with 0.5% caseine and 0.01% merthiolate pH 7.5) was added. After incubation for 1 hour at 37°C the cells were washed with 0.05% Tween-20 in PBS containing 0.01% merthiolate (washing buffer), after which the Fv-fragment samples were added (any dilutions in blocker). After shaking for 2 hours at 37°C the material that was not bound was washed away and 100 µl of a 1 µg/ml anti-TAG1 antibody in blocker was added to every well. Subsequently the plate was again shaken for two hours and washed three times. After that a biotinylated sheep anti-mouse-Ig-antibody was added (Amersham, Buckinghamshire, GB, 100 µl of a 1/1000 dilution). After one hour shaking at 37°C and three times washing the reaction was finished with the biotinylated peroxidase-streptavidin complex, H₂O₂ and ABTS.

Because no anti-idiotype antibody was available the detection was performed by means of the TAG1-polypeptide that was linked to the V_{K}-region.

The highest expression of Fv in the supernatant of a 18 hours incubated cell culture amounted to 30 binding units. This means that a 1/30 dilution of the fluid was still positive (green colour) in the ELISA for HuIFN-Γ binding. Almost no Fv was demonstrated in the periplasma for the same induction. This is caused by the fact that the Fv-concentration in the periplasma reaches its maximal value after an induction of two hours after which the Fv leaks away into the culture liquid and after 18 hours is found substantially therein. This "leakage" out of the cells could be due to a stress situation in the bacteria caused by a high production of a strange protein. In the fluid of cells that were not induced (absence of IPTG) a basal amount of Fv was always present while the fluid of cells with a suppressed promotor, because of the presence of glucose, contained no detectable Fv-material.

The periplasma preparation method provided already a substantial enrichment of the produced Fv because the volume was much smaller in comparison to that of the supernatant. The periplasmic fraction of one liter culture amounted to 12 ml (83 times more concentrated) and of a 1 to 3 ml culture this amounted 75 µl (13 to 40 times more concentrated). After a two hours induction with 0.1 mM IPTG (the combination giving the highest induction in the periplasma) the fluid as well as the periplasmic fraction were tested in the ELISA. The periplasma had a titer of 200 binding units; in the supernatant no Fv was detected.

From these experiments it is clear that E. coli, wherein the Fv-gen was introduced produced indeed Fv and that the Fv was capable of binding HuIFN-Γ. This means that neither the aminoacid alterations in the 5'- and 3'-ends of teh V-regions nor the linking of TAG1 to V_{K} have an important negative influence on the binding of the Fv of D9D10 to the HuIFN-Γ-molecule.

### EXAMPLE 6

### Inhibition of the antiviral activity of HuIFN-Γ

To verify whether the Fv-fragment produced by E. coli can inhibit or neutralize the activity of HuIFN-Γ in vitro neutralization tests were performed. For these A549-cells were used, a human carcinoma cell line. This cell line was cultured in MEM with 10% "Newborn Calf Serum" (NCS) and is sensitive to the antiviral activity of HuIFN-Γ.

A human-IFN-Γ being just antivirally active (167 ng HuIFN-Γ with a specific activity of 2.8 x 10⁴U/µg on Wish cell; Biogen, Gent) were mixed with serial 1/2 logarithmic dilutions of the samples to be tested. These were incubated for 4 hours at 37°C after which the mixtures were desinfected for 6 minutes under UV light. To all the wells 50,000 A549 cells were added and the whole was incubated for 20 hours in a CO₂-incubator until the cells had formed a mono-layer. The infection was performed with a 1/300 dilution of the Encephalo Myocarditis Virus (EMCV). After 48 hours, when the virus control was totally affected, the living cells were coloured with 100 µl of a neutral red solution (1/30 dilution neutral red in PBS with Ca²+ and Mg²+) and stored for 2 to 4 hours in the dark. Subsequently they were washed with PBS (with Ca²+ and Mg²+) and 100 µl acidified alcohol was added to every well. After 20 minutes the plates were read in a Multiscan Titertek at 542 nm.

As controls the samples in the absence of HuIFN-Γ and IFN-Γ in the absence of the sample were used. Cell- and virus controls were also tested.

A 1/3 dilution of this fraction appeared to be capable to block the antiviral activity completely. When a combination of the 1/3 dilution together with a low IFN-Γ dose was brought on the cell the EMC-virus could infect and kill the cells, contrary to the sample with the low IFN-Γ concentration alone, with which protection occurred. This inhibiting effect was not due to a possible toxicity of a component from the periplasma because in the absence of virus the cells were not affected, neither to a stimulation of the virus growth, because the cells were not faster affected in the presence of the periplasmic fraction than in the absence thereof. In a 1/6 dilution of the periplasmic fraction no inhibition of the antiviral activity was observed. The Fv-fragment that was purified on a affinity column showed the same neutralizing effects. This indicates that it is no other component from the periplasma that blocks the antiviral activity of HuIFN-Γ.

Moreover periplasma of non-induced cells was not capable of neutralizing. This means that the Fv of the anti-HuIFN-Γ antibody of D9D10 is also capable of neutralizing the antiviral activity of HuIFN-Γ, despite of minor changes in the 5' and 3'-ends of the V-regions and the linking of TAG1 to V_{K}.

The above examples show that the introduction of the V_{H}-V_{L} PCR-fragments behind the LacZ-promoter provides for a dicistronic mRNA being formed which produces the F_{H}-F_{L}, proteins separately but probably in equal amounts. Upstream from every V-gene a ribosome binding site is present. Since the the *pel*B signal peptide provides the transport of the pectate lyase enzyme from the cytoplasma to periplasm both V-proteins will be transported the periplasm. This means that the Fv-fragment can easily be isolated and obtained in a concentrated form. It is then also not subject to the degradation by cytoplasmic proteases.

As was shown by ELISA and neutralization tests the functional HuIFN-Γ binding Fv-fragment was secreted into the periplasm. The Fv-fragment, binding the HuIFN-Γ molecule and neutralising its anti-viral activity, and only consisting of both variabel regions and a small polypeptide (TAG1) will probaly have a reduced HAMA response as compared to the parental antibody. The absence of the C-regions has no further influence on a good immunoresponse because no effect or functions are necessary for this antibody.

The stability of the Fv-fragment can be increased by chemical cross-linking of the V-regions, by introduction of an intermoleculair disulphidebridge or by coupling of the V-regions with a protein linker. Coupling of a toxine, cytotoxic substance or radio-isotope to the Fv-fragment can be performed in case it is used as a magic bullet. this coupling also increases the half life in the serum.

Formule Ia :
PstI
CAG GTC CAG CTG CAG GAG TCT GGA CCG GAG CTG AAG AAG CCT GGA
GAG ACA GTC AAG ATC TCC TGC AAG GCT TCT GGG TAC ACC TTC ACA
GAC TAT GGA ATG AAC TGG GTG AAG CAG GCT CCA GGA CAG GGT TTA
AAG TGG ATG GGC TGG ATA AAC ACC TAC ACT GGA GAG TCA ACA TAT
GTT GAT GAC TTC AAG GGA CGC TTT GTC TTC TCT TTG GAA ACC TCT
GCC AGT GCT GCC TAT TTG CAG ATC AAC AAC CTC AAA AAT GAG GAC
ACG GCT ACA TAT TTC TGT GCA AGA AGG GGT TTT TAT GCT ATG GAC
TAC TGG GGC CAA GGG ACC ACG GTC ACC GTC TCC TCA
BstEII

Formule Ib :
SacI
GAC ATC GAG CTC ACC CAG TCT CCA GCA ATC ATG TCT GCA TCT CCA
GGG GAG AAG GTC ACC TTG ACC TGC AGT GCC AGC TCA AGT ATA AGT
TAC ATG TTC TGG TAT CAC CAG AGG CCA GGA TCC TCC CCC AGA CTC
CTG ATT TAT GAC ACA TCC AAC CTG GCT TCT GGA GTC CCT GTT CGC
TTC AGT GGC AGT GGG TCT GGG ACC TCT TAC TCT CTC ACA ATC AGC
CGA ATG GAG GCT GAA GAT GCT GCC ACT TAT TTC TGC CAT CAG TCG
AGT AGT TAC CCA TTC ACG TTC GGC TCG GGG ACC AAG CTC GAG ATC
AAA
XhoI

Formule Ic :
GGT GGA GGC GGT TCA GGC GGA GGT GGC TCT GGC GGT GGC GGA TCG

## Claims

1. A recombinant DNA-molecule for the expression of a functional Fv-fragment of a desired antibody, directed towards interferon, comprising a homologous promoter sequence, a homologous terminator sequence and dicistronic heterologous DNA-sequence, the cistrons of which encoding the variable part of a heavy chain (V_{H}) of the desired anti-interferon antibody and the variable part of a light chain (V_{K}) of the desired anti-interferon antibody, respectively.

2. A recombinant DNA-molecule as claimed in claim 1, characterized in that the cistrons of the heterologous DNA-sequence encode the variable part of the heavy chain, and light chain of an antibody directed towards human interferon-γ, respectively.

3. A recombinant DNA-molecule as claimed in claim 2, characterized in that the cistrons are comprised in the following nucleotide sequences, or mutated versions thereof: 
formula Ia
PstI
CAG GTC CAG CTG CAG GAG TCT GGA CCG GAG CTG AAG AAG CCT GGA
GAG ACA GTC AAG ATC TCC TGC AAG GCT TCT GGG TAC ACC TTC ACA
GAC TAT GGA ATG AAC TGG GTG AAG CAG GCT CCA GGA CAG GGT TTA
AAG TGG ATG GGC TGG ATA AAC ACC TAC ACT GGA GAG TCA ACA TAT
GTT GAT GAC TTC AAG GGA CGC TTT GTC TTC TCT TTG GAA ACC TCT
GCC AGT GCT GCC TAT TTG CAG ATC AAC AAC CTC AAA AAT GAG GAC
ACG GCT ACA TAT TTC TGT GCA AGA AGG GGT TTT TAT GCT ATG GAC
TAC TGG GGC CAA GGG ACC ACG GTC ACC GTC TCC TCA
BstEII
 or

formula Ib
SacI
GAC ATC GAG CTC ACC CAG TCT CCA GCA ATC ATG TCT GCA TCT CCA
GGG GAG AAG GTC ACC TTG ACC TGC AGT GCC AGC TCA AGT ATA AGT
TAC ATG TTC TGG TAT CAC CAG AGG CCA GGA TCC TCC CCC AGA CTC
CTG ATT TAT GAC ACA TCC AAC CTG GCT TCT GGA GTC CCT GTT CGC
TTC AGT GGC AGT GGG TCT GGG ACC TCT TAC TCT CTC ACA ATC AGC
CGA ATG GAG GCT GAA GAT GCT GCC ACT TAT TTC TGC CAT CAG TCG
AGT AGT TAC CCA TTC ACG TTC GGC TCG GGG ACC AAG CTC GAG ATC
AAA
XhoI

4. Recombinant DNA-molecule as claimed in claim 3, characterized in that the cistron encoding the variable part of the light chain is represented by the formula Ib and the cistron encoding the heavy chain is represented by formula Ia, or by mutated versions thereof that do not adversely affect the functionality of the Fv-fragment.

5. Recombinant DNA-molecule as claimed in any one of the claims 1-4, characterized in that the homologous promoter sequence is the lac-promoter.

6. Recombinant DNA-molecule as claimed in any one of the preceding claims, characterized in that the recombinant DNA-molecule comprises a signal peptide sequence upstream from each of the cistrons and a sequence encoding the TAG1-polypeptide downstream of the second cistron.

7. A recombinant DNA-molecule as claimed in any one of the claims 1-5, characterized in that the cistrons of the heavy and light chain, respectively are connected by means of a linker sequence as represented by formula Ic, that upstream of the cistron encoding the heavy chain a signal peptide sequence is comprised and that downstream of the cistron encoding the light chain the sequence encoding the TAG1-polypeptide is comprised.

8. A recombinant DNA-molecule as claimed in claim 6 or 7, characterized in that the signal peptide sequence is derived from pelB.

9. E.coli strain comprising a recombinant DNA-molecule as claimed in any one of the preceding claims.

10. E.coli strain as claimed in claim 9, characterized in that the strain expresses the Fv-fragment of an antibody directed towards human-interferon-γ.

11. E.coli strain as claimed in claim 10, characterized in that the strain expresses the Fv-fragment of the monoclonal anti-human-interferon-γ antibody characterized by V_{H}- and V_{K}-nucleotide sequences comprised respectively in formula Ia and Ib as defined in claim 3, or mutated versions thereof.

12. A method for preparing a recombinant DNA-molecule as claimed in any one of the preceding claims, comprising the steps of:
a) linearizing a DNA-molecule comprising the homologous promoter and terminator regions to obtain a linear DNA-molecule;
b) isolating and multiplying a variable part of a heavy chain (V_{H}-fragment) of a desired antibody;
c) ligating the linear DNA-molecule and the V_{H}-fragment to obtain a first recombinant DNA-molecule;
d) linearizing the first recombinant DNA-molecule to obtain a first linear DNA-molecule;
e) isolating and multiplying a variable part of a light chain (V_{K}-fragment) of a desired antibody; and
f) ligating the linearized first DNA-molecule and the V_{K}-fragment to obtain a second recombinant DNA-molecule.

13. A method as claimed in claim 12, characterized in that the desired DNA-fragments are multiplied by means of Polymerase Chain Reaction.

14. A method as claimed in claim 12 or 13, characterized in that the V_{K}-fragment has a DNA-sequence comprised in the sequence as represented in formula Ib as defined in claim 3, or mutated versions thereof.

15. A method according to any one of the claims 12 to 14, characterized in that the V_{H}-fragment has a DNA-sequence comprised in the sequence as represented in formula Ia as defined in claim 3, or mutated versions thereof.

16. A method according to any one of the claims 12 to 15, characterized in that between the sequences for the heavy and light chain, respectively, a linker sequence comprised in the sequence as represented in formula Ic: GGT GGA GGC GGT TCA GGC GGA GGT GGC TCT GGC GGT GGC GGA TCG or mutated versions thereof, is cloned.

17. A method for preparing a recombinant Fv-fragment of a desired antibody, comprising:
a) producing a recombinant DNA-molecule as claimed in any one of the claims 1 to 8;
b) transforming a host cell culture with the recombinant DNA-molecule to obtain a recombinant host cell culture;
c) culturing the host cell culture under conditions allowing the expression of the DNA-sequence encoding the recombinant Fv-fragment; and
d) isolating the recombinant Fv-fragment.

18. A method as claimed in claim 17, characterized in that the recombinant Fv-fragment is the Fv-fragment of the monoclonal anti-human-interferon-γ antibody characterized by V_{H}- and V_{K}-nucleotide sequences comprised respectively in formula Ia and Ib as defined in claim 3, or mutated versions thereof.

19. A method as claimed one of the claims 17 or 18, characterized in that the host cell is E.coli.

20. A recombinant Fv-fragment of an antibody directed towards human interferon-γ, prepared according to the method as claimed in any one of the claims 17-19.

21. A biological active preparation comprising an antihuman interferon-γ Fv-fragment as claimed in claim 20.

22. A biological active preparation as claimed in claim 21 having a neutralizing effect on the antiviral activity of human interferon-γ.

23. Biological active preparation as claimed in claim 21 or 22 for use in the treatment of pathological reactions caused by interferon-γ.

24. Biological active preparation as claimed in claim 21 or 22 for use in the treatment of endotoxic shock, local inflammation, cerebral malaria, allergic encephalomyelitis, skin allograft rejection, auto-immune arthritis, tumor development and lupus-like disease.

## Patentansprüche

1. Rekombinates DNA-Molekül zur Expression eines funktionellen Fv-Fragments eines gewünschten Antikörpers, der auf Interferon gerichtet ist, das eine homologe Promotorsequenz, eine homologe Terminatorsequenz und eine dicistronische heterologe DNA-Sequenz enthält, deren Cistrons den variablen Teil einer schweren Kette (V_{H}) des gewünschten anti-Interferon-Antikörpers bzw. den variablen Teil einer leichten Kette (V_{K}) des gewünschten anti-Interferon Antikörpers kodieren.

2. Rekombinates DNA-Molekül nach Anspruch 1, dadurch gekennzeichnet, daß die Cistrons der heterologen DNA-Sequenz den variablen Teil der schweren Kette bzw. der leichten Kette eines auf Humaninterferon-γ gerichteten Antikörpers kodieren.

3. Rekombinates DNA-Molekül nach Anspruch 2, dadurch gekennzeichnet, daß die Cistrons in den folgenden Nukleotidsequenzen enthalten sind oder mutierte Versionen davon: 
Formel Ia

PstI
CAG GTC CAG CTG CAG GAG TCT GGA CCG GAG CTG AAG AAG CCT GGA
GAG ACA GTC AAG ATC TCC TGC AAG GCT TCT GGG TAC ACC TTC ACA
GAC TAT GGA ATG AAC TGG GTG AAG CAG GCT CCA GGA CAG GGT TTA
AAG TGG ATG GGC TGG ATA AAC ACC TAC ACT GGA GAG TCA ACA TAT
GTT GAT GAC TTC AAG GGA CGC TTT GTC TTC TCT TTG GAA ACC TCT
GCC AGT GCT GCC TAT TTG CAG ATC AAC AAC CTC AAA AAT GAG GAC
ACG GCT ACA TAT TTC TGT GCA AGA AGG GGT TTT TAT GCT ATG GAC
TAC TGG GGC CAA GGG ACC ACG GTC ACC GTC TCC TCA
BstEII
 oder

Formel Ib

SacI
GAC ATC GAG CTC ACC CAG TCT CCA GCA ATC ATG TCT GCA TCT CCA
GGG GAG AAG GTC ACC TTG ACC TGC AGT GCC AGC TCA AGT ATA AGT
TAC ATG TTC TGG TAT CAC CAG AGG CCA GGA TCC TCC CCC AGA CTC
CTG ATT TAT GAC ACA TCC AAC CTG GCT TCT GGA GTC CCT GTT CGC
TTC AGT GGC AGT GGG TCT GGG ACC TCT TAC TCT CTC ACA ATC AGC
CGA ATG GAG GCT GAA GAT GCT GCC ACT TAT TTC TGC CAT CAG TCG
AGT AGT TAC CCA TTC ACG TTC GGC TCG GGG ACC AAG CTC GAG ATC
AAA
XhoI

4. Rekombinates DNA-Molekül nach Anspruch 3, dadurch gekennzeichnet, daß das den variablen Teil der leichten Kette kodierende Cistron durch die Formel Ib dargestellt wird und das die schwere Kette kodierende Cistron durch die Formel Ia dargestellt wird, oder durch mutierte Versionen davon, die die Funktionalität des Fv-Fragments nicht nachteilig beeinflussen.

5. Rekombinates DNA-Molekül nach einem der Ansprüche 1-4, dadurch gekennzeichnet, daß die homologe Promotorsequenz der lac-Promotor ist.

6. Rekombinates DNA-Molekül nach einem der vorstehenden Ansprüche, dadurch gekennzeichnet, daß das rekombinate DNA-Molekül stromaufwärts von jedem der Cistrons eine Signalpeptidsequenz und stromabwärts von dem zweiten Cistron eine das TAG1-Polypeptid kodierende Sequenz enthält.

7. Rekombinates DNA-Molekül nach einem der Ansprüche 1-5, dadurch gekennzeichnet, daß die Cistrons der schweren und leichten Kette jeweils durch eine durch Formel Ic dargestellte Linkersequenz verbunden sind, daß stromaufwärts des die schwere Kette kodierenden Cistrons eine Signalpeptidsequenz enthalten ist und daß stromabwärts des die leichte Kette kodierenden Cistrons die das TAG1-Polypeptid kodierende Sequenz enthalten ist.

8. Rekombinates DNA-Molekül nach Anspruch 6 oder 7, dadurch gekennzeichnet, daß die Signalpeptidsequenz von pelB stammt.

9. E.coli Stamm, der ein rekombinates DNA-Molekül nach einem der vorstehenden Ansprüche umfaßt.

10. E.coli Stamm nach Anspruch 9, dadurch gekennzeichnet, daß der Stamm das Fv-Fragment eines auf menschliches Interferon-γ gerichteten Antikörpers exprimiert.

11. E.coli Stamm nach Anspruch 10, dadurch gekennzeichnet, daß der Stamm das Fv-Fragment des monoclonalen anti-Humaninterferon-γ Antikörpers exprimiert, der durch V_{H}- und V_{K}-Nucleotidsequenzen gekennzeichnet ist, die jeweils in Formel Ia und Ib nach Anspruch 3 enthalten sind, oder mutierte Versionen davon.

12. Verfahren zur Herstellung eines rekombinanten DNA-Moleküls nach einem der vorstehenden Ansprüche, das die folgenden Schritte umfaßt:
a) Linearisieren eines DNA-Moleküls, das die homologen Promotor- und Terminatorbereiche enthält, um ein lineares DNA-Molekül zu erhalten;
b) Isolieren und Vermehren eines variablen Teils einer schweren Kette (V_{H}-Fragment) eines gewünschten Antikörpers;
c) Ligieren des linearen DNA-Moleküls und des V_{H}-Fragments, um ein erstes rekombinates DNA-Molekül zu erhalten;
d) Linearisieren des ersten rekombinaten DNA-Moleküls, um ein erstes lineares DNA-Molekül zu erhalten;
e) Isolieren und Vermehren eines variablen Teils einer leichten Kette (V_{K}-Fragment) eines gewünschten Antikörpers; und
f) Ligieren des linearisierten ersten DNA-Moleküls und des V_{K}-Fragments, um ein zweites rekombinates DNA-Molekül zu erhalten.

13. Verfahren nach Anspruch 12, dadurch gekennzeichnet, daß die gewünschten DNA-Fragmente durch die Polymerase Kettenreaktion vermehrt werden.

14. Verfahren nach Anspruch 12 oder 13, dadurch gekennzeichnet, daß das V_{K}-Fragment eine DNA-Sequenz aufweist, die in der in Formel Ib gemäß Anspruch 3 dargestellten Sequenz enthalten ist, oder mutierte Versionen davon.

15. Verfahren nach einem der Ansprüche 12 bis 14, dadurch gekennzeichnet, daß das V_{H}-Fragment eine DNA-Sequenz aufweist, die in der in Formel Ia gemäß Anspruch 3 dargestellten Sequenz enthalten ist, oder mutierte Versionen davon.

16. Verfahren nach einem der Ansprüche 12 bis 15, dadurch gekennzeichnet, daß zwischen den Sequenzen für die schwere bzw. leichte Kette eine in der durch Formel Ic: GGT GGA GGC GGT TCA GGC GGA GGT GGC TCT GGC GGT GGC GGA TCG dargestellten Sequenz enthaltene Linkersequenz oder mutierte Versionen davon kloniert wird.

17. Verfahren zur Herstellung eines rekombinaten Fv-Fragments eines gewünschten Antikörpers, umfassend:
a) Herstellen eines rekombinaten DNA-Moleküls nach einem der Ansprüche 1 bis 8;
b) Transformieren einer Wirtszellenkultur mit dem rekombinaten DNA-Molekül, um eine rekombinate Wirtszellenkultur zu erhalten;
c) Züchten der Wirtszellenkultur unter Bedingungen, die die Expression der das rekombinante Fv-Fragment kodierenden DNA-Sequenz ermöglichen; und
d) Isolieren des rekombinanten Fv-Fragments.

18. Verfahren nach Anspruch 17, dadurch gekennzeichnet, daß das rekombinante Fv-Fragment das Fv-Fragment des monoclonalen anti-Humaninterferon-γ Antikörpers ist, der durch V_{H}- und V_{K}-Nucleotidsequenzen gekennzeichnet ist, die jeweils in Formel Ia und Ib gemäß Anspruch 3 enthalten sind, oder mutierte Versionen davon.

19. Verfahren nach einem der Ansprüche 17 oder 18, dadurch gekennzeichnet, daß die Wirtszelle E.coli ist.

20. Rekombinates Fv-Fragment eines auf menschliches Interferon-y gerichteten Antikörpers, das nach einem in einem der Ansprüche 17-19 beanspruchten Verfahren hergestellt wird.

21. Biologisch aktives Präparat, das ein anti-Humaninterferon-γ Fv-Fragment nach Anspruch 20 enthält.

22. Biologisch aktives Präparat nach Anspruch 21, das eine neutralisierende Wirkung auf die antivirale Aktivität von menschlichem Interferon-γ aufweist.

23. Biologisch aktives Präparat nach Anspruch 21 oder 22 zur Verwendung bei der Behandlung von pathologischen Reaktionen, die durch Interferon-γ verursacht werden.

24. Biologisch aktives Präparat nach Anspruch 21 oder 22 zur Verwendung bei der Behandlung von endotoxischem Schock, einer lokalen Entzündung, cerebraler Malaria, allergischer Encephalomyelitis, Hautallotransplantatabstoßung, Autoimmun-Arthritis, Tumorentwicklung und lupusartigen Krankheiten.

## Revendications

1. Molécule d'ADN recombinant pour l'expression d'un fragment Fv fonctionnel d'un anticorps désiré, dirigé contre l'interféron, comprenant une séquence promotrice homologue, une séquence terminatrice homologue et une séquence d'ADN dicistronique herétologue, dont les cistrons codent respectivement pour la partie variable d'une chaîne lourde (V_{H}) de l'anticorps anti-interféron désiré et pour la partie variable d'une chaîne légère (V_{K}) de l'anticorps anti-interféron désiré.

2. Molécule d'ADN recombinant selon la revendication 1, caractérisée en ce que les cistrons de la séquence d'ADN hétérologue codent respectivement pour la partie variable de la chaîne lourde et la chaîne légère d'un anticorps dirigé contre l'interféron γ humain.

3. Molécule d'ADN recombinant selon la revendication 2, caractérisée en ce que les cistrons sont compris dans les séquences de nucléotides suivantes ou dans des versions mutées de celles-ci : 
formule Ia

PstI
CAG GTC CAG CTG CAG GAG TCT GGA CCG GAG CTG AAG AAG CCT GGA
GAG ACA GTC AAG ATC TCC TGC AAG GCT TCT GGG TAC ACC TTC ACA
GAC TAT GGA ATG AAC TGG GTG AAG CAG GCT CCA GGA CAG GGT TTA
AAG TGG ATG GGC TGG ATA AAC ACC TAC ACT GGA GAG TCA ACA TAT
GTT GAT GAC TTC AAG GGA CGC TTT GTC TTC TCT TTG GAA ACC TCT
GCC AGT GCT GCC TAT TTG CAG ATC AAC AAC CTC AAA AAT GAG GAC
ACG GCT ACA TAT TTC TGT GCA AGA AGG GGT TTT TAT GCT ATG GAC
TAC TGG GGC CAA GGG ACC ACG GTC ACC GTC TCC TCA
BstEII
 ou

formule Ib

SacI
GAC ATC GAG CTC ACC CAG TCT CCA GCA ATC ATG TCT GCA TCT CCA
GGG GAG AAG GTC ACC TTG ACC TGC AGT GCC AGC TCA AGT ATA AGT
TAC ATG TTC TGG TAT CAC CAG AGG CCA GGA TCC TCC CCC AGA CTC
CTG ATT TAT GAC ACA TCC AAC CTG GCT TCT GGA GTC CCT GTT CGC
TTC AGT GGC AGT GGG TCT GGG ACC TCT TAC TCT CTC ACA ATC AGC
CGA ATG GAG GCT GAA GAT GCT GCC ACT TAT TTC TGC CAT CAG TCG
AGT AGT TAC CCA TTC ACG TTC GGC TCG GGG ACC AAG CTC GAG ATC
AAA
XhoI

4. Molécule d'ADN recombinant selon la revendication 3, caractérisée en ce que le cistron qui code pour la partie variable de la chaîne légère est représenté par la formule Ib et le cistron qui code pour la chaîne lourde est représenté par la formule Ia, ou des versions mutées de celles-ci qui n'affectent pas d'une façon contraire la fonctionnalité du fragment Fv.

5. Molécule d'ADN recombinant selon l'une quelconque des revendications 1 à 4, caractérisée en ce que la séquence promotrice homologue est le promoteur lac.

6. Molécule d'ADN recombinant selon l'une quelconque des revendications précédentes, caractérisée en ce que la molécule d'ADN recombinant comprend une séquence peptide signal en amont de chacun des cistrons et une séquence codant pour le polypeptide TAG1 en aval du second cistron.

7. Molécule d'ADN recombinant selon l'une quelconque des revendications 1 à 5, caractérisée en ce que les cistrons de la chaîne lourde et légère, respectivement, sont reliés au moyen d'une séquence liante telle que représentée par la formule Ic, en ce que en amont du cistron codant pour la chaîne lourde est comprise une séquence peptide signal et en ce que en aval du cistron codant pour la chaîne légère est comprise la séquence codant pour le polypeptide TAG1.

8. Molécule d'ADN recombinant selon la revendication 6 ou 7, caractérisée en ce que la séquence du peptide signal est dérivée de peIB.

9. Souche de E. coli comprenant une molécule d'ADN recombinant selon l'une quelconque des revendications précédentes.

10. Souche de E. coli selon la revendication 9, caractérisée en ce que cette souche exprime le fagment Fv d'un anticorps dirigé contre l'interféron γ humain.

11. Souche de E. coli selon la revendication 10, caractérisée en ce que la souche exprime le fragment Fv de l'anticorps monoclonal anti-interféron γ humain caractérisé par des séquences nucléotidiques V_{H} et V_{K} comprises respectivement dans les séquences de formules Ia et Ib telles que définies dans la revendication 3, ou des versions mutées de celles-ci.

12. Méthode pour la préparation d'une molécule d'ADN recombinant selon l'une quelconque des revendications précédentes, comprenant les étapes de :
a) linéarisation d'une molécule d'ADN comprenant les régions promotrices et terminatrices homologues pour obtenir une molécule d'ADN linéaire;
b) isolement et multiplication d'une partie variable d'une chaîne lourde (fragment V_{H}) d'un anticorps désiré;
c) ligature de la molécule d'ADN linéaire et du fragment V_{H} pour obtenir une première molécule d'ADN recombinant;
d) linéarisation de la première molécule d'ADN recombinant pour obtenir une première molécule d'ADN linéaire;
e) isolement et multiplication d'une partie variable d'une chaîne légère (fragment V_{K}) d'un anticorps désiré; et
f) ligature de la première molécule d'ADN linéarisée et du fragment V_{K} pour obtenir une seconde molécule d'ADN recombinant.

13. Méthode selon la revendication 12, caractérisée en ce que les fragments d'ADN désirés sont multipliés par une réaction de polymérisation en chaîne (PCR "Polymerase Chain reaction").

14. Méthode selon l'une quelconque des revendications 12 ou 13, caractérisée en ce que le fragment V_{K} a une séquence d'ADN comprise dans la séquence représentée par la formule Ib telle que définie dans la revendication 3, ou des version mutées de celle-ci.

15. Méthode selon l'une quelconque des revendications 12 à 14, caractérisée en ce que le fragment V_{H} a une séquence d'ADN comprise dans la séquence telle que représentée dans la formule la définie dans la revendication 3, ou des versions mutées de celle-ci.

16. Méthode selon l'une quelconque des revendications 12 à 15, caractérisée en ce que entre les séquences de la chaîne lourde et la chaîne légère, respectivement, est clonée une séquence liante comprise dans la séquence représentée dans la formule Ic : GGT GGA GGC GGT TCA GGC GGA GGT GGC TCT GGC GGT GGC GGA TCG ou des versions mutées de celle-ci.

17. Méthode pour la préparation d'un fragment Fv recombinant d'un anticorps désiré, comprenant :
a) la production d'une molécule d'ADN recombinant selon l'une quelconque des revendications 1 à 8;
b) la transformation d'une cellule hôte en culture avec la molécule d'ADN recombinant pour obtenir une cellule hôte recombinante en culture;
c) la culture de la cellule hôte dans des conditions permettant l'expression de la séquence d'ADN codant pour le fragment Fv recombinant; et
d) l'isolement du fragment Fv recombinant.

18. Méthode selon la revendication 17, caractérisée en ce que le fragment Fv recombinant est le fragment Fv de l'anticorps monoclonal anti-interféron γ humain caractérisé par des séquences nucléotidiques V_{H} et V_{K} comprises respectivement dans les formules Ia et Ib définies dans la revendication 3, ou des versions mutées de celles-ci.

19. Méthode selon l'une quelconque des revendications 17 ou 18, caractérisée en ce que la cellule hôte est E. coli.

20. Fragment Fv recombinant d'un anticorps dirigé contre l'interféron γ humain, préparé selon la méthode telle que revendiquée dans l'une quelconque des revendications 17 à 19.

21. Préparation biologique active comprenant un fragment Fv anti-interféron γ humain selon la revendication 20.

22. Préparation biologique active selon la revendication 21 ayant un effet neutralisant sur l'activité antivirale de l'interféron γ humain.

23. Préparation biologique active selon la revendication 21 ou 22 pour une utilisation dans le traitement de réactions pathologiques causées par l'interféron γ humain.

24. Préparation biologique active selon la revendication 21 ou 22 pour une utilisation dans le traitement de chocs endotoxiques, d'inflammation locale, de malaria cérébrale, d'encéphalomyélite allergique, de rejet d'allogreffe de peau, d'arthrite auto-immune, de développement de tumeur et de maladies de type lupus.
